# FASCICULE DE BREVET EUROPEEN

(11) **EP 3 253 885 B1**
(45) Date de publication et mention de la délivrance du brevet: **02.01.2019**
(21) Numéro de dépôt: 16707181.0
(22) Date de dépôt: 04.02.2016
(51) Int. Cl.: C12Q 1/04, C12N 1/20, C12Q 1/24

(54) **ENRICHISSEMENT ET CULTURE SÉLECTIVE DE MYCOBACTÉRIES**
ANREICHERUNG UND SELEKTIVE KULTUR VON MYKOBAKTERIEN
ENRICHMENT AND SELECTIVE CULTURE OF MYCOBACTERIA

(30) Priorité: 06.02.2015 FR 1550959
(43) Date de publication de la demande: 13.12.2017
(73) Titulaire: bioMérieux, 69280 Marcy-l'Étoile (FR)
(72) Inventeur: ORENGA, Sylvain, 01160 Neuville sur Ain (FR); PERRY, Audrey, Heaton Newcastle upon Type NE7 7BH (GB); PERRY, John, High Heaton Newcastle upon Tyne NE7 7BH (GB); PREECE, Clair, Walker Newcastle upon Type NE6 4AX (GB)
(86) Numéro de dépôt international: PCT/FR2016/050239
(87) Numéro de publication internationale: WO 2016/124863

(56) Documents cités:
- US-A- 4 263 398
- US-A1- 2014 199 724
- VERMIS K ET AL: "Isolation of Burkholderia cepacia Complex Genomovars from Waters", SYSTEMATIC AND APPLIED MICROBIOLOGY, URBAN & FISCHER, AMSTERDAM, NL, vol. 26, no. 4, 1 janvier 2003 (2003-01-01), pages 595-600, XP004957352, ISSN: 0723-2020, DOI: 10.1078/072320203770865909 cité dans la demande
- LAINE L ET AL: "A novel chromogenic medium for isolation of Pseudomonas aeruginosa from the sputa of cystic fibrosis patients", JOURNAL OF CYSTIC FIBROSIS, ELSEVIER, NL, vol. 8, no. 2, 1 mars 2009 (2009-03-01), pages 143-149, XP025962622, ISSN: 1569-1993, DOI: 10.1016/J.JCF.2008.11.003 [extrait le 2009-02-23]
- MAUREEN E CAMPBELL ET AL: "New Selective Medium for Pseudomonas aeruginosa with Phenanthroline and 9-Chloro-9-[4-(Diethylamino)Phenyl]-9,10- Dihydro-10-Phenylacridine Hydrochloride (C-390)", JOURNAL OF CLINICAL MICROBIOLOGY, vol. 26, 1 septembre 1988 (1988-09-01), pages 1910-1912, XP055219980, cité dans la demande
- C. R. ESTHER ET AL: "Detection of Rapidly Growing Mycobacteria in Routine Cultures of Samples from Patients with Cystic Fibrosis", JOURNAL OF CLINICAL MICROBIOLOGY, vol. 49, no. 4, 1 avril 2011 (2011-04-01), pages 1421-1425, XP055219962, US ISSN: 0095-1137, DOI: 10.1128/JCM.02379-10 cité dans la demande

## Description

La présente invention a trait aux méthodes de culture et d'isolement des mycobactéries. Plus précisément, elle concerne les méthodes de microbiologie et les milieux de culture pouvant être utilisés pour la détection, l'identification, l'isolement et/ou l'étude analytique de mycobactéries, par exemple celles présentes dans les échantillons et les prélèvements biologiques.

Appartenant à l'ordre des actinomycètes, les mycobactéries sont des bacilles fins, de forme droite ou parfois légèrement incurvée. Du fait d'une paroi riche en lipides qui limite la pénétration des colorants, la coloration des mycobactéries par les techniques et les colorants traditionnels est difficile. Mais une fois colorées, leur coloration résiste à la décoloration aussi bien par l'acide que par l'alcool. Pour cette raison, elles sont qualifiées de bactéries « acido-alcoolo-résistantes ».

Selon une classification élémentaire, les différentes espèces de mycobactéries sont répertoriées selon deux grandes catégories.

La première catégorie regroupe les mycobactéries pathogènes strictes, qui parasitent aussi bien les animaux que l'homme. Ces mycobactéries sont responsables de la tuberculose (*Mycobacterium tuberculosis, Mycobacterium bovis, Mycobacterium africanum, Mycobacterium microti et Mycobacterium canetti*) ou de la lèpre (*Mycobacterium leprae*).

La seconde catégorie regroupe les mycobactéries saprophytes ou commensales, qui sont habituellement non-pathogènes. On parle alors de mycobactéries « atypiques » ou « non-tuberculeuses » (MNT). Ubiquitaires dans notre environnement, ces dernières se trouvent disséminées un peu partout dans le sol, les végétaux, la poussière, l'eau et les réseaux de distribution, notre alimentation... Généralement inoffensives pour l'homme, certaines mycobactéries non-tuberculeuses peuvent causer, chez les sujets immunodéprimés, des infections opportunistes atteignant principalement les poumons, la peau et le système lymphatique.

Ainsi, sur le plan clinique mais aussi sanitaire, qu'elles soient pathogènes ou non-tuberculeuses, la détection et/ou l'identification des mycobactéries revêtent une importance substantielle. Une détection précoce et spécifique de ces bactéries permet de proposer une solution adaptée, en termes de traitement thérapeutique, de décontamination sanitaire... Différentes techniques existent à cet égard, par exemple, l'amplification génique (par PCR) de séquences nucléiques spécifiques, l'immuno-détection d'antigènes, la spectrométrie de masse, la détection microbiologique d'activités enzymatiques...

Quelles qu'elles soient, ces méthodes de détection/identification nécessitent généralement la mise en culture des mycobactéries éventuellement présentes dans les échantillons/prélèvements à analyser, pour obtenir une quantité propice à leur détection. Quelles qu'elles soient, ces méthodes détection/identification souffrent d'un même handicap : la lenteur de croissance et de développement des mycobactéries.

En effet, comparées à d'autres bactéries, aux levures et aux champignons, les mycobactéries, aussi bien pathogènes que saprophytes, se caractérisent par une croissance et un développement relativement lents. Par ailleurs, dans les échantillons biologiques dans lesquels la présence de mycobactéries est recherchée, les mycobactéries se trouvent rarement isolées ; elles sont usuellement contaminées par une flore accompagnatrice dont la croissance et le développement sont beaucoup plus rapides. Au fur des cycles de divisions cellulaires, la proportion des mycobactéries dans l'échantillon décroit, et la difficulté de pouvoir les visualiser et les identifier s'en trouve accentuée.

Pour remédier à cette lenteur de croissance et de développement et/ou à cette contamination par d'autres microorganismes, de nombreux milieux de culture et bouillons d'enrichissement, plus ou moins sélectifs pour les mycobactéries ou pour une espèce particulière de mycobactérie, ont été développés.

Parmi les plus fréquemment utilisés, le milieu Löwenstein-Jensen est un milieu de culture coagulé (non gélosé), comprenant une composante nutritive à base d'oeuf entier, de fécule de pomme de terre, d'asparagine, de glycérol et de minéraux. Ce milieu contient aussi du vert malachite, en vue d'inhiber la flore accompagnatrice (plus particulièrement, les bactéries à Gram négatif). Une adjonction de pénicilline et/ou d'acide nalidixique a également été proposée pour en augmenter la sélectivité à l'égard des mycobactéries.

De composition similaire, le milieu gélosé Coletsos renferme en plus, du pyruvate et du glutamate, du bleu de tournesol et de la gélatine.

Plus récemment apparus, les milieux Middlebrook 7H9, 7H10 et 7H11 sont des milieux semi-synthétiques ayant une composante nutritive à base d'acides aminés, d'acides gras, de pyruvate et de sels minéraux. Ces milieux contiennent également de la catalase et de l'albumine bovine (fraction V), qui apportent aux mycobactéries une protection contre divers agents nocifs (notamment des peroxydes toxiques) éventuellement présents dans les échantillons et prélèvements biologiques. Le milieu Middlebrook 7H10 diffère du milieu Middlebrook 7H9, par la présence de vert malachite. Le milieu Middlebrook 7H11 diffère du milieu Middlebrook 7H10 en ce qu'il est enrichi avec une peptone pancréatique de caséine. De nombreuses variantes à ces trois milieux Middlebrook ont aussi été envisagées pour en augmenter la sélectivité à l'égard des mycobactéries, par exemple en y ajoutant les agents sélectifs suivant : polymyxine, amphotéricine B, acide nalidixique, triméthoprime, azlocilline et vancomycine.

Dernièrement, le milieu BCSA (pour « *Burkholderia cepacia* selective agar »), initialement développé pour la culture et la détection de *Burkholderia cepacia,* a été décrit comme étant particulièrement propice au développement et à la croissance des mycobactéries, plus particulièrement des mycobactéries dites à croissance rapide (Esther et al. - Journal of Clinical Microbiology ; 2011, 1421-1425). Il s'agit là d'un milieu de culture gélosé, formulé à partir d'une composante nutritive comprenant de la peptone de caséine, des sucres (lactose et saccharose), un extrait de levure, et des agents sélectifs tels que la gentamicine, la vancomycine, le cristal violet et la polymyxine B.

La demande US20140199724 décrit un milieu de culture pour l'enrichissement et la détection de mycobactéries.

La présente invention vise à améliorer les méthodes et techniques utilisées pour détecter, identifier et/ou isoler les mycobactéries présentes dans un échantillon biologique. Pour ce faire, la présente invention vise à améliorer les milieux de culture et bouillons d'enrichissement mis en oeuvre dans ces méthodes et techniques, tant au niveau de leur fertilité que de leur sélectivité à l'égard des mycobactéries. Egalement, elle vise à proposer de nouvelles compositions de milieu de culture et/ou de bouillon d'enrichissement, aptes à permettre la culture et la multiplication des mycobactéries, et ayant des performances améliorées en termes de fertilité et/ou de sélectivité à l'égard des mycobactéries.

Avant de présenter l'invention, les définitions suivantes sont données pour permettre une meilleure compréhension de l'invention.

Par « milieu de culture », on entend un milieu comprenant tous les éléments nécessaires à l'expression d'un métabolisme et/ou à la croissance de microorganismes. Le milieu de culture peut être solide, semi-solide ou liquide. Par « milieu solide », on entend par exemple un milieu gélifié ou un milieu coagulé. L'agar est l'agent gélifiant traditionnel en microbiologie pour la culture des microorganismes, mais il est possible d'utiliser de la gélatine, de l'agarose ou d'autres gélifiants naturels ou artificiels.

Par « bouillon d'enrichissement », on désigne plus spécifiquement un milieu de culture liquide.

Un milieu de culture est qualifié de milieu minimum, lorsque sa composition comporte uniquement les éléments chimiques strictement nécessaires à la croissance et la multiplication des microorganismes à cultiver. On y trouve classiquement :
- de l'eau (généralement, de l'eau distillée ou désionisée) ;
- une composante nutritive comprenant généralement :
   - une source carbonée et d'énergie (généralement du glucose) ;
   - une source de calcium (par exemple CaCl₂,) ;
   - une source d'azote (par exemple (NH₄)₂SO₄) ;
   - une source de soufre (par exemple (NN₄)₂SO₄) ;
   - une source de magnésium (par exemple MgCl₂) ;
   - une source de fer (par exemple du citrate de fer) ;
   - une source d'oligo-éléments (par exemple sels de Cu, Zn, Co, Ni, B, Ti) ;
- éventuellement un tampon pH pour maintenir le milieu à un pH approprié ; et
pour les milieux solides ou semi-solides, éventuellement un agent gélifiant (par exemple l'agar, la gélatine ou l'agarose).

L'adjonction de facteurs de croissance particuliers et/ou de nutriments divers permet de renforcer les attributs de la composante nutritive et d'accroitre le pouvoir fertilisant du milieu. On parle alors de milieu « enrichi ». L'adjonction de ces facteurs de croissance et/ou de ces nutriments peut être réalisée au moyen de composés ou de compositions chimiquement définis, ou alors au moyen de compositions complexes (par exemple, sang frais, sérums, extrait de levure, oeuf entier, jaune d'oeuf, peptones...).

Un milieu de culture est dit sélectif lorsqu'il comprend au moins un agent sélectif permettant audit milieu de favoriser la croissance d'un microorganisme-cible ou d'un groupe-cible de microorganismes, plutôt que celle de la flore accompagnatrice. Il s'agit essentiellement de composés aux effets antibiotiques et/ou antifongiques, et présentant une spécificité de toxicité (toxicité plus faible pour les microorganismes-cibles que pour la flore accompagnatrice).

Par « échantillon biologique », on entend un échantillon clinique issu d'un prélèvement d'origine humaine ou animale, ou un échantillon alimentaire issu de tout type d'aliment. Cet échantillon biologique peut être liquide ou solide et on peut citer d'une manière non limitative, un échantillon clinique de sang, de plasma, d'urines, de fèces, des prélèvements de nez, de gorges, de peaux, de plaies, de liquide céphalo-rachidien, de liquide broncho-alvéolaire, d'expectoration, un échantillon alimentaire d'eau, de boissons tels que le lait, un jus de fruits, de yaourt, de viande, d'oeufs, de légumes, de mayonnaise, de fromage, de poisson..., un échantillon alimentaire issu d'une alimentation destinée aux animaux, tel que notamment un échantillon issu de farines animales. A des fins de simplification de vocabulaire, on utilisera indifféremment les expressions « échantillon biologique » et « prélèvement biologique ».

La présente invention concerne ainsi un procédé (une méthode) d'enrichissement et de culture sélective de mycobactéries contenues dans un échantillon biologique, dans lequel on ensemence tout ou partie dudit échantillon dans/sur un milieu de culture comprenant une composante nutritive propice au développement et à la croissance de mycobactéries, caractérisé en ce que ledit milieu de culture comprend également, à titre d'agents sélectifs, au moins l'hydrochlorure de 9-chloro-9-(4'-diéthylamino)phényl-9,10-dihydro-10-phénylacridine et un agent apte à inhiber des bactéries *Pseudomonas aeruginosa.*

La présente invention repose sur le principe général d'incorporer, dans des compositions de milieux de culture envisagés pour la culture de mycobactéries, le 9-chloro-9-(4'-diéthylamino)phényl-9,10-dihydro-10-phénylacridine, pour en accroitre la sélectivité à l'égard des mycobactéries. Cet agent sélectif est aussi connu sous la dénomination générique C-390 (CAS 77769-31-4). Jusqu'alors essentiellement utilisé à des fins de culture sélective de *Pseudomonas aeruginosa* (US 4,263,398 et Campbell et al. - Journal of Clinical Microbiology ; 1988, 1910-1912) ou d'isolement de génomovars du complexe *Burkholderia cepacia* (Vermis et al., - Systematic and Applied Microbiology, 2003 (26) 595-600), les inventeurs ont démontré que l'utilisation d'un tel composé dans les milieux de culture pouvait également conduire à un bon isolement des mycobactéries et une bonne élimination de la flore accompagnatrice, notamment des microorganismes appartenant aux genres *Pseudomonas, Enterobacteriaceae, Streptococcus, Burkholderia, Stenotrophomonas, Staphylococcus, Enterococcus, Achromobacter, Acinetobacter, Pandoraea, Bacillus, Elizabethkingia miricola, Haemophilus, Moraxella, Neisseria, Aspergillus, Scedosporium, Candida* et *Geosmithia.*

Egalement, les inventeurs ont constaté que le C-390 conférait aussi aux milieux de culture, une légère teinte bleutée supplémentaire qui peut améliorer notablement la visualisation et la détection visuelle des colonies poussant sur ces milieux.

Avantageusement et selon l'invention, la concentration en C-390 du milieu de culture utilisé pour mettre en oeuvre le procédé d'enrichissement et de culture sélective selon l'invention est comprise entre 2 mg/L et 1000 mg/L, préférentiellement entre de l'ordre de 4 mg/L et de l'ordre de 256 mg/L, et plus préférentiellement encore entre de l'ordre de 8 mg/L et de l'ordre de 128 mg/L.

A titre d'exemples d'agents aptes à inhiber des bactéries *Pseudomonas aeruginosa,* on peut citer notamment le méthanesulfonate de colistine, la fosfomycine, l'acide nalidixique, l'aztréonam, la cefsulodine et le mangrolide A.

Avantageusement et selon l'invention, on peut utiliser également dans ledit milieu de culture, un agent apte à inhiber les bactéries *Burkholderia cepacia,* préférentiellement un agent choisi parmi la fosfomycine, l'aztréoname et le mangrolide A.

Selon un mode préféré de mise en oeuvre de l'invention, ledit milieu de culture comprend en outre, au moins un agent sélectif additionnel, choisi parmi :
- l'amphotéricine B, notamment à une concentration préférentiellement comprise entre 1 mg/L et 200 mg/L, et plus préférentiellement encore entre 2 mg/L et 50 mg/L ;
- l'acide nalidixique, notamment à une concentration préférentiellement comprise entre 5 mg/L et 200 mg/L, et plus préférentiellement encore entre 15 mg/L et 100 mg/L ;
- la vancomycine, notamment à une concentration préférentiellement comprise entre 1 mg/L et 50 mg/L, et plus préférentiellement encore entre 2 mg/L et 20 mg/L ;
- le méthanesulfonate de colistine, notamment à une concentration préférentiellement comprise entre 10 mg/L et 200 mg/L, et plus préférentiellement encore entre 15 mg/L et 150 mg/L ;
- la fosfomycine, notamment à une concentration préférentiellement comprise entre 100 mg/L et 3000 mg/L, et plus préférentiellement encore entre 200 mg/L et 2 000 mg/L ; et
- le vert de malachite, notamment à une concentration préférentiellement comprise entre 0,05 mg/L et 2 mg/L, et plus préférentiellement encore entre 0,2 mg/L et 1 mg/L.

Selon un mode préféré de l'invention, ledit milieu de culture comprend à titre d'agents sélectifs additionnels :
- l'amphotéricine B, à une concentration comprise entre 2 mg/L et 50 mg/L,
- le méthanesulfonate de colistine, à une concentration comprise entre 15 mg/L et 150 mg/L,
- la fosfomycine, à une concentration comprise entre 200 mg/L et 2 000 mg/L.

Selon un mode préféré de l'invention, en complément de la fosfomycine, ledit milieu de culture comprend avantageusement du glucose-6-phosphate, notamment à une concentration préférentiellement comprise entre 10 mg/L et 50 mg/L.

Selon un aspect particulier, la présente invention vise à améliorer les méthodes de culture et/ou d'isolement de mycobactérie qui ont actuellement cours, en intervenant sur la composition des milieux de culture usuels (tels que des milieux Middlebrook, le milieu Löwenstein-Jensen, le milieu Columbia, le milieu BCSA) pour en accroitre la sélectivité en inhibant les non-mycobactéries. A cet effet, la présente invention propose de parfaire la composition de ces milieux de culture par un apport de C-390, et éventuellement d'autres agents sélectifs comme précédemment énoncés. Egalement, la présente invention propose d'en améliorer la fertilité grâce à l'apport de divers nutriments tels que : le glycérol, un supplément à base d'acide oléique, d'albumine, de dextrose et de catalase (ou supplément OADC), le charbon actif, le sang frais, un extrait de levure, l'alpha-cétoglutarate, la caséine, le pyruvate, des peptones, l'amarante, le jaune d'oeuf, de l'ARN, le polyoxyéthylène stéarate, le tyloxapol.

Dans ce contexte, le procédé d'enrichissement et de culture sélective de mycobactéries selon l'invention est avantageusement mis en oeuvre avec un milieu de culture dont la composante nutritive reprend celle d'un milieu de culture choisi parmi :
- un milieu Middlebrook (en particulier un des milieux Middlebrook 7H9, 7H10 et 7H11) éventuellement complémenté avec du glycérol et/ou un supplément OADC ;
- un milieu Löwenstein-Jensen ;
- un milieu Columbia, éventuellement complémenté avec un supplément OADC et/ou du sang frais, notamment du sang de cheval ; et
- un milieu BCSA.

Selon un mode encore plus préféré, le procédé d'enrichissement et de culture sélective de mycobactéries selon l'invention est mis en oeuvre avec un milieu de culture comprenant une composante nutritive reprenant celle d'un milieu Middlebrook, complémentée avec :
- du glycérol, notamment à une concentration préférentiellement comprise entre 2 mL/L et 20 mL/L,
- un supplément OADC, notamment à une concentration préférentiellement comprise entre 50 mL/L et 200 mL/L, et éventuellement
- un ou plusieurs nutriments choisis parmi : le sang frais, un extrait de levure, l'alpha-cétoglutarate, la caséine, le pyruvate, des peptones, l'amarante, le jaune d'oeuf, de l'ARN, le polyoxyéthylène stéarate, le tyloxapol.

Avantageusement et selon l'invention, ledit supplément OADC est une composition aqueuse comprenant 50 g/L d'albumine bovine, 20 g/L de glucose, 0,04 g/L de catalase et 0,5 g/L d'acide oléique.

Avantageusement et selon ce mode préféré de l'invention, le procédé d'enrichissement et de culture sélective de mycobactéries selon l'invention est mis en oeuvre avec un milieu de culture comprenant une composante nutritive reprenant celle d'un milieu Middlebrook, complémentée avec :
- du glycérol, notamment à une concentration préférentiellement comprise entre 2 mL/L et 20 mL/L,
- un supplément OADC, notamment à une concentration préférentiellement comprise entre 50 mL/L et 200 mL/L, et
- un extrait de levure, notamment à une concentration préférentiellement comprise entre 0,1 g/L et 20 g/L.

Avantageusement et selon l'invention, ledit procédé s'applique plus particulièrement à l'enrichissement et à la culture sélective des mycobactéries appartenant aux espèces *M. abscessus, M. bolletii, M. massiliense, M. chelonae, M. immunogenum, M. salmoniphilum, M. avium, M. tuberculosis, M. intracellulare, M. malmoense, M. gordonae, M. kansasii et M. fortuitum.*

Avantageusement et selon l'invention, les colonies de mycobactéries se développant sur ledit milieu de culture sont visuellement repérées en utilisant des substrats synthétiques chromogéniques et/ou fluorogéniques spécifiques d'activités enzymatiques exprimées par les mycobactéries recherchées. A cet effet, on peut citer le 4-méthylumbelliféryl*-beta*-D-glucoside qui permet le marquage des colonies exprimant une activité *beta*-glucosidase.

La présente invention s'étend également à un milieu de culture adapté à la mise en oeuvre d'un procédé d'enrichissement et de culture sélective de mycobactéries selon l'invention. En l'espèce, un milieu de culture selon l'invention comprend une composante nutritive propice au développement et à la croissance de mycobactéries, et est caractérisé en ce qu'il comprend également, à titre d'agents sélectifs, de l'hydrochlorure de 9-chloro-9-(4'-diéthylamino)phényl-9,10-dihydro-10-phénylacridine et un agent apte à inhiber des bactéries *Pseudomonas aeruginosa*, et à titre d'agents sélectifs additionnels :
- l'amphotéricine B, à une concentration comprise entre 2 mg/L et 50 mg/L ;
- le méthane sulfonate de colistine, à une concentration comprise entre 15 mg/L et 150 mg/L ;
- la fosfomycine, à une concentration comprise entre 200 mg/L et 2000 mg/L, et du glucose-6-phosphate, à une concentration comprise entre 10 mg/L et 50 mg/L.

Selon un mode particulier de réalisation, ledit milieu de culture est exempt de cristal violet.

Est aussi décrit un milieu de culture comprenant une composante nutritive propice au développement et à la croissance de mycobactéries et, à titre d'agents sélectifs, de l'hydrochlorure de 9-chloro-9-(4'-diéthylamino)phényl-9,10-dihydro-10-phénylacridine et un agent apte à inhiber des bactéries *Pseudomonas aeruginosa.* Ledit milieu de culture se caractérise également par tout ou partie des caractéristiques techniques suivantes :
- la concentration en C-390 est comprise entre 2 mg/L et 1000 mg/L, préférentiellement entre de l'ordre de 4 mg/L et de l'ordre de 256 mg/L, et plus préférentiellement encore entre de l'ordre de 8 mg/L et de l'ordre de 128 mg/L ;
- ledit milieu de culture comprend au moins un agent sélectif additionnel (autre que C-390 et l'agent choisi pour inhiber *Pseudomonas aeruginosa*), choisi parmi :
   - l'amphotéricine B, notamment à une concentration préférentiellement comprise entre 1 mg/L et 200 mg/L, et plus préférentiellement encore entre 2 mg/L et 50 mg/L ;
   - l'acide nalidixique, notamment à une concentration préférentiellement comprise entre 5 mg/L et 200 mg/L, et plus préférentiellement encore entre 15 mg/L et 100 mg/L ;
   - la vancomycine, notamment à une concentration préférentiellement comprise entre 1 mg/L et 50 mg/L, et plus préférentiellement encore entre 2 mg/L et 20 mg/L ;
   - le méthanesulfonate de colistine, notamment à une concentration préférentiellement comprise entre 10 mg/L et 200 mg/L, et plus préférentiellement encore entre 15 mg/L et 150 mg/L ; et
   - la fosfomycine, notamment à une concentration préférentiellement comprise entre 100 mg/L et 3000 mg/L, plus préférentiellement encore entre 200 mg/L et 2000 mg/L, et éventuellement du glucose-6-phosphate, notamment à une concentration préférentiellement comprise entre 10 mg/L et 50 mg/L ;
- ledit milieu de culture comprend à titre d'agents sélectifs additionnels :
   - l'amphotéricine B, à une concentration comprise entre 2 mg/L et 50 mg/L ;
   - le méthanesulfonate de colistine, à une concentration comprise entre 15 mg/L et 150 mg/L ;
   - la fosfomycine, à une concentration comprise entre 200 mg/L et 2000 mg/L, et du glucose-6-phosphate, notamment à une concentration préférentiellement comprise entre 10 mg/L et 50 mg/L ;
- ledit milieu de culture comprend une composante nutritive reprenant celle d'un milieu de culture choisi parmi :
   - un milieu Middlebrook, éventuellement complémenté avec du glycérol et/ou un supplément OADC ;
   - un milieu Löwenstein-Jensen ;
   - un milieu Columbia, éventuellement complémenté avec un supplément OADC et/ou du sang frais, notamment du sang de cheval ; et
   - un milieu BCSA ;
- ledit milieu de culture comprend une composante nutritive reprenant celle d'un milieu Middlebrook, complémentée avec :
   - du glycérol, notamment à une concentration préférentiellement comprise entre 2 mL/L et 20 mL/L,
   - un supplément OADC, notamment à une concentration préférentiellement comprise entre 50 mL/L et 200 mL/L, et éventuellement
   - un ou plusieurs nutriments choisis parmi : le sang frais, un extrait de levure, l'alpha-cétoglutarate, la caséine, le pyruvate, des peptones, l'amarante, le jaune d'oeuf, de l'ARN, le polyoxyéthylène stéarate, le tyloxapol.

Avantageusement, la présente invention porte sur un milieu de culture comprenant une composante nutritive consistant en milieu Middlebrook 7H9, complémentée avec :
- du glycérol, à une concentration comprise entre 2 mL/L et 20 mL/L,
- un supplément OADC, à une concentration comprise entre 50 mL/L et 200 mL/L, et
- un extrait de levure, à une concentration comprise entre 0,1 g/L et 20 g/L ;
et comprenant à titre d'agents sélectifs :
- du C-390, à une concentration comprise entre 2 mg/L et 50 mg/L,
- de l'amphotéricine B, à une concentration comprise entre 2 mg/L et 50 mg/L,
- du méthanesulfonate de colistine, à une concentration comprise entre 15 mg/L et 150 mg/L,
- de la fosfomycine, à une concentration comprise entre 200 mg/L et 2000 mg/L, accompagnée de glucose-6-phosphate, à une concentration comprise entre 10 mg/L et 50 mg/L.

Selon un mode avantageux de mise en oeuvre de l'invention, la composition dudit milieu de culture peut inclure un ou plusieurs marqueurs aptes à permettre une détection et/ou une identification visuelle des mycobactéries se développant dans ou sur ledit milieu. Il peut s'agir notamment de substrats enzymatiques synthétiques, aux propriétés chromogéniques et/ou fluorogéniques.

Ce document concerne également une méthode, un procédé d'enrichissement et de culture sélective de mycobactéries, une utilisation du C-390 à des fins de culture et/ou d'isolement de mycobactéries, ainsi qu'un milieu de culture, caractérisés par tout ou partie de caractéristiques techniques exposées ci-dessus et ci-dessous.

D'autres buts, caractéristiques et avantages de l'invention apparaîtront au vu de la description qui va suivre et des exemples développés ci-dessous, qui ont pour but de faciliter la compréhension de l'invention et de sa mise en oeuvre. Ces exemples sont donnés à titre explicatif et ne sauraient limiter la portée de l'invention.

### EXEMPLES : Elaboration et évaluation de milieux sélectifs selon l'invention

### 1/ - Sélection d'une composition « de base » pour la culture des mycobactéries

Différentes compositions de milieux de culture, connues de la littérature comme fertiles aux mycobactéries, ont été testées et comparées pour leur capacité à faire croître les mycobactéries. Certains ont été enrichis au préalable. Les milieux testés sont les suivants :
- milieu A : agar Middlebrook,
- milieu B : agar Midllebrook enrichi en glycérol (4 mL/L),
- milieu C : agar Middlebook avec du Tween 80 (1 g/L),
- milieu D : agar Midllebrook enrichi à la fois avec du glycérol (4 mL/L) et un supplément OADC (100 mL/L),
- milieu E : agar Midllebrook complémenté/enrichi à la fois avec du Tween 80 et le supplément OADC,
- milieu F : gélose Columbia (Oxoid),
- milieu G : gélose Columbia (Oxoid), enrichie avec le supplément OADC,
- milieu H : agar Columbia au sang,
- milieu I : milieu BCSA (bioMérieux, France).

### a) Préparation des milieux A à E

Les milieux référencés A à E ont été préparés à partir d'une solution-mère consistant en un bouillon de culture Middlebrook 7H9 dix fois concentré (Middlebrook 7H9 10X), ayant pour composition :
- sulfate d'ammonium (5 g/L),
- acide L-glutamique (5 g/L),
- phosphate disodique (25 g/L),
- phosphate monopotassique (10 g/L),
- citrate de sodium (1 g/L),
- sulfate de magnésium (0,5 g/L),
- chlorure de calcium (0,005 g/L),
- sulfate de zinc (0,01 g/L),
- sulfate de cuivre (0,01 g/L),
- ammonium ferrique citrate (0,4 g/L),
- pyridoxine (0,01 g/L), et
- biotine (0,005 g/L).

Le pH de ce bouillon a été ajusté à 6,6 ± 0,2. Le bouillon a ensuite été stérilisé à l'autoclave à 116°C pendant 10 minutes. Il est conservé au réfrigérateur jusqu'à utilisation.

Pour obtenir le milieu A, 50 mL de bouillon ont été ajoutés à 450 mL d'eau désionisée. 5 g d'agar bactériologique sont ajoutés à l'ensemble pour obtenir un agar Middlebrook. Le tout a ensuite été stérilisé à l'autoclavage à 116°C, pendant 10 minutes.

Les milieux B, C, D et E ont été préparés de façon similaire au milieu A, et renferment en plus :
- du glycérol (4 mL/L) et éventuellement un supplément à base d'acide oléique, d'albumine, de dextrose et de catalase (supplément OADC ; 100 mL/L),
ou bien :
- du Tween 80 (1 g/L) et éventuellement un supplément OADC (100 mL/L).

### b) Milieux F à I

Les milieux F, H et I sont des milieux disponibles dans le commerce.

Le milieu G a été préparé de façon similaire au milieu F, et renferme en plus le supplément OADC (100 mL/L).

### c) Evaluation de la fertilité des milieux A à I à l'égard des mycobactéries

La fertilité de chacun des milieux A à I a été évaluée au moyen de 20 souches de mycobactérie :
- 12 souches de *Mycobacterium abscessus,*
- 3 souches de *Mycobacterium bolletii,*
- 5 souches de *Mycobacterium massiliense.*

Ces souches, initialement conservées à -20°C dans le glycérol, sont décongelées et cultivées, dans un premier temps sur gélose Columbia au sang de cheval (5 %). Chaque souche est alors mise en suspension dans 1 mL de solution saline (0,85%) jusqu'à atteindre une turbidité équivalente au standard McFarland 0,5 (équivalent à environ 1,5.10⁸ CFU/mL).

Pour les mycobactéries ayant tendance à s'agglutiner, les cellules sont dispersées au vortex, dans un tube contenant 3 billes de verre de 3 mm de diamètre, pendant 10 minutes.

Une aliquote de 1 µL de chaque suspension de mycobactéries est inoculée sur chacun des milieux de culture à évaluer, et étalée de façon à obtenir des colonies isolées. Les cultures sont incubées à 30°C. La croissance des colonies est observée pendant 7 jours.

Les résultats obtenus sont résumés dans le Tableau 1 ci-après.

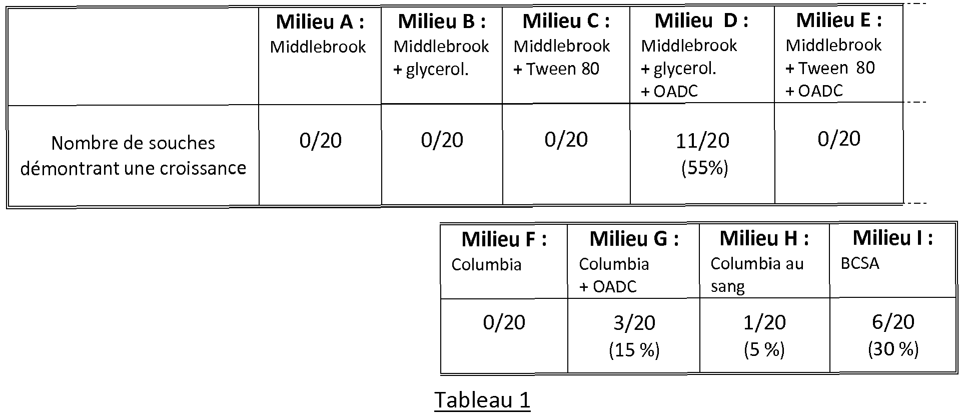

### 2/ - Evaluation de l'effet de divers nutriments sur la croissance des mycobactéries

Sur la base de la formulation du milieu D précédemment testée, les effets de divers nutriments sur la culture des mycobactéries ont été évalués : charbon actif, sang de cheval, extrait de levure, alpha-cétoglutarate, caséine, pyruvate, peptones, amarante, émulsion de jaune d'oeuf, ARN (SIGMA, réf. R6625-25G), polyoxyéthylène stéarate, tyloxapol.

De manière très similaire au milieu D, les milieux suivant (noté J à V) ont été préparés :

| | |
|---|---|
| - Milieu J : | Milieu D + charbon actif (2 g/L), |
| - Milieu K : | Milieu D + sang de cheval 5 % (50 mL/L), |
| - Milieu L : | Milieu D + extrait de levure (4 g/L), |
| - Milieu M : | Milieu D + alpha-cétoglutarate (15 g/L), |
| - Milieu N : | Milieu D + caséine (1 g/L), |
| - Milieu O : | Milieu D + pyruvate de sodium (7 g/L), |
| - Milieu P : | Milieu D + peptone (10 g/L), |
| - Milieu K : | Milieu D + amarante (0,04 g/L), |
| - Milieu R : | Milieu D + émulsion de jaune d'oeuf (33 mL/L), |
| - Milieu S : | Milieu D + ARN (0,05 g/L), |
| - Milieu T : | Milieu D + polyoxyéthylène stéarate (0,1 g/L) |
| - Milieu U : | Milieu D + tyloxapol (5 mL/L) |
| - Milieu V : | Milieu D + émulsion de jaune d'oeuf (33 mL/L) + charbon actif (4 g/L) |

La fertilité de ces milieux est évaluée au moyen des 20 mêmes souches de mycobactéries utilisées dans le test précédemment décrit, et traitées de façon identique avant ensemencement.

Les cultures sont incubées à 30°C. La croissance des colonies est suivie pendant 7 jours. Le Tableau 2 ci-après résume les constatations faites aux 3^{ème} et 7^{ème} jours de culture.

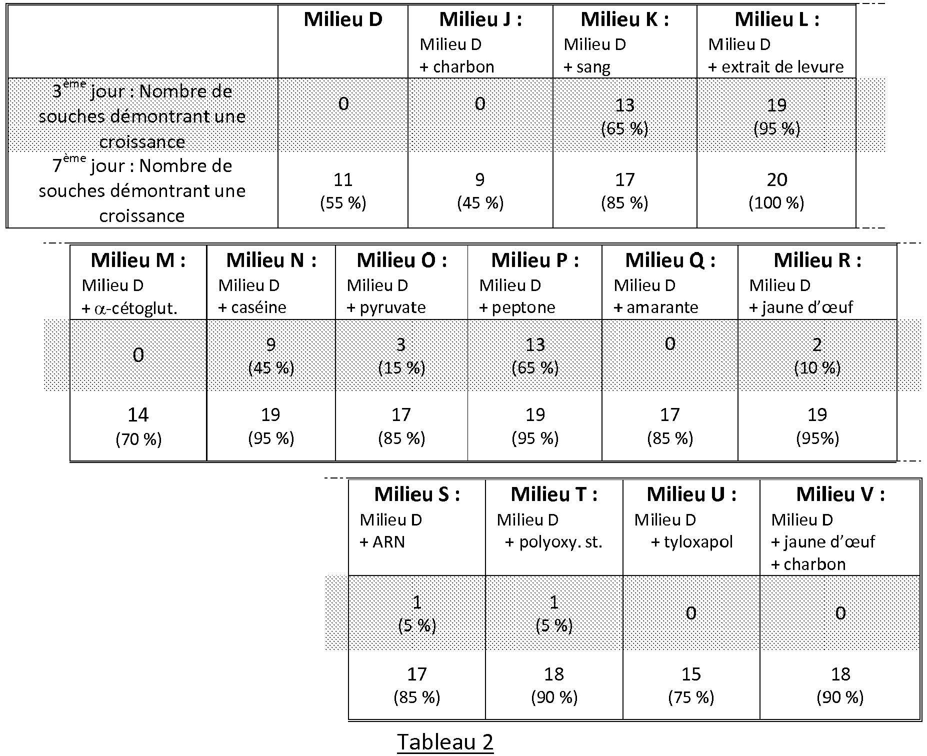

### 3/ - Evaluation de l'effet de différents agents sélectifs à l'égard des mycobactéries

Sur la base de la formulation du Milieu L précédemment développée et testée, les effets de différents agents sélectifs ont été évalués pour leur niveau de sélectivité à l'égard des mycobactéries : amphotéricine B (un antifongique), acide nalidixique, vancomycine, méthanesulfonate de colistine, C-390 (BIOSYNTH, Suisse), fosfomycine et vert malachite, à différentes concentrations.

Le tableau 3 ci-après résume les résultats de cette évaluation, réalisée sur les 20 souches de mycobactérie utilisées dans les tests précédemment décrits, et sur 95 souches de microorganismes n'appartenant pas au genre *Mycobacterium,* après 7 jours d'incubation. Parmi les 95 souches évoquées, on compte :
- 50 souches de *Pseudomonas aeruginosa,*
- 10 autres souches de bactéries non-fermentaires :
   - 1 souche *d'Achromobacter xylosoxidans,*
   - 1 souche d'*Acinetobacter* sp.,
   - 1 souche de *Burkholderia multivorans,*
   - 1 souche de *Burkholderia cenocepacia,*
   - 1 souche de *Burkholderia cepacia,*
   - 1 souche de *Burkholderia contaminans,*
   - 1 souche de *Pandoraea apista,*
   - 1 souche de *Pandoraea pnomenusa,*
   - 2 souches de *Stenotrophomonas maltophilia,*
- 7 souches d'entérobactéries :
   - 1 souche d'*Escherichia coli,*
   - 1 souche de *Klebsiella pneumoniae,*
   - 1 souche de *Providentia rettgeri,*
   - 1 souche d'*Enterobacter cloacae,*
   - 1 souche *d'Enterobacter aerogenes,*
   - 1 souche de *Serratia marcescens,*
   - 1 souche de *Citrobacterfreundii,*
- 10 souches d'espèces de bactérie à Gram positif :
   - 2 souches de *Staphylococcus aureus,*
   - 1 souche de *Staphylococcus epidermidis,*
   - 1 souche de *Streptococcus pyogenes,*
   - 1 souche de *Streptococcus pneumoniae,*
   - 1 souche de *Streptococcus salivarius,*
   - 1 souche de *Streptococcus gordonii,*
   - 1 souche d'*Enterococcus faecium,*
   - 1 souche d'*Enterococcus faecalis,* et
   - 1 souche de *Bacillus subtilis.*
- 8 souches de champignons/levures :
   - 1 souche de *Candida albicans,*
   - 1 souche de *Candida glabrata,*
   - 2 souches d'*Aspergillus fumigatus,*
   - 1 souche *d'Aspergillus terreus,*
   - 1 souche de *Scedosporium apiospermum,*
   - 1 souche de *Scedosporium prolificans,*
   - 1 souche de *Rasamsonia* (*Geosmithia*) *argillacea.*

Dans ce Tableau 3, les résultats sont présentés en nombre et en pourcentage de souches ayant démontré une croissance sur le milieu considéré après 7 jours de culture. Les mentions NS et NT signifient respectivement « non significatif » (inhibition de bactéries par l'amphotéricine B) et « non testé » (action des antibactériens sur les levures et champignons).

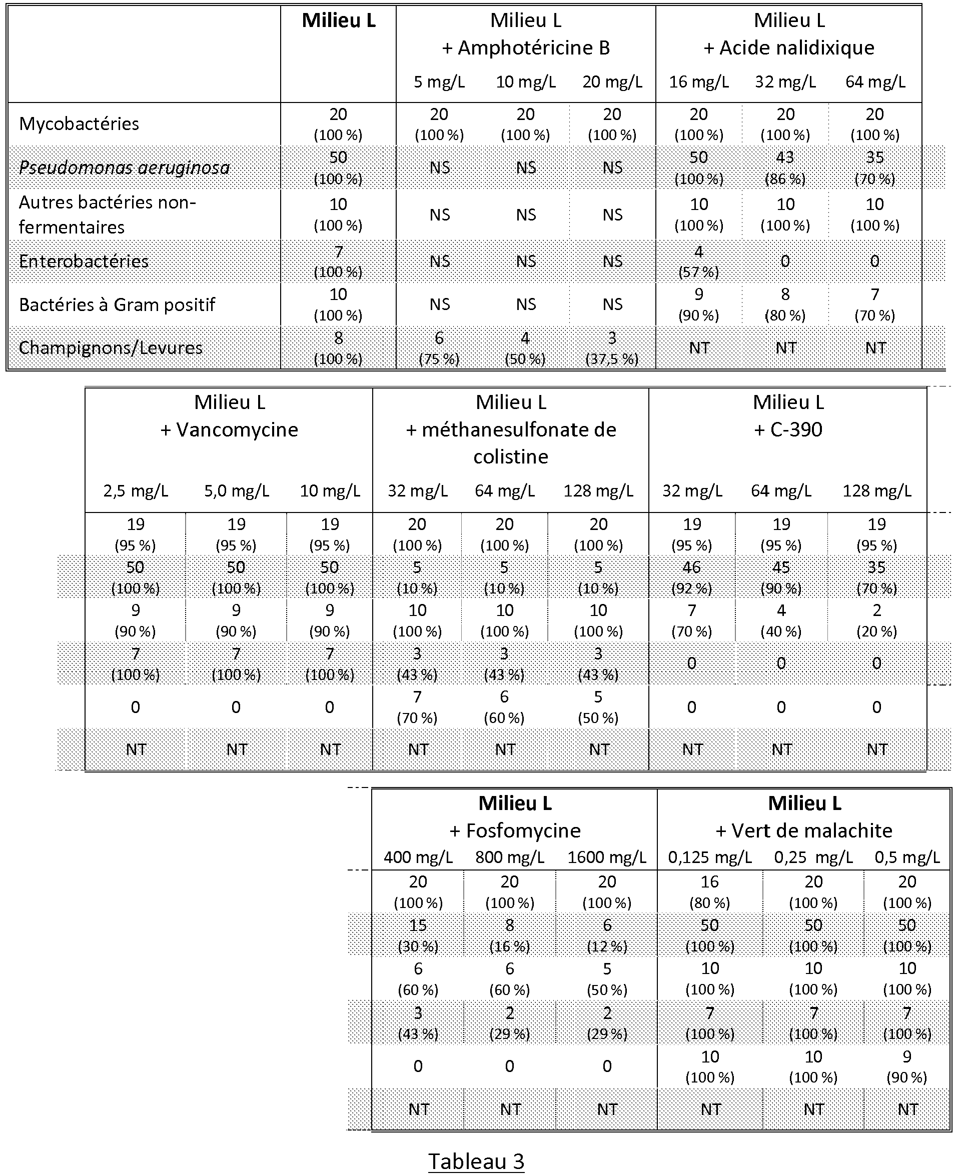

### 4/ - Optimisation des milieux sélectifs pour mycobactéries

Au vu des données précédentes, deux nouveaux milieux de culture sélectifs, notés Milieu W et Milieu X, ont été mis au point. Ces milieux ont été développés à partir de la composition du Milieu L, un milieu de culture enrichi tel que précédemment décrit, à laquelle une combinaison particulière d'agents sélectifs a été ajoutée. La composition desdits milieux sélectifs est résumée comme suit :

| | |
|---|---|
| - Milieu W : | Milieu L + méthanesulfonate de colistine (32 mg/L) |
| | + fosfomycine (400 mg/L) + glucose-6-phosphate (25 mg/L) |
| | + Amphotéricine B (5 mg/L) |
| - Milieu X (Milieu selon l'invention) : | |
| | Milieu L + méthanesulfonate de colistine (32 mg/L) |
| | + fosfomycine (400 mg/L) + glucose-6-phosphate (25 mg/L) |
| | + Amphotéricine B (5 mg/L) |
| | + C-390 (32 mg/L). |

Ces deux milieux ont été testés sur 188 souches de microorganismes :
- 98 souches de mycobactéries non-tuberculeuses :
   - 52 souches de *Mycobacterium abscessus,*
   - 3 souches de *Mycobacterium bolletii,*
   - 34 souches de *Mycobacterium chelonae,*
   - 1 souche de *Mycobacterium immunogenum,*
   - 6 souches de *Mycobacterium massiliense,*
   - 2 souches de *Mycobacterium salmoniphilum,*
- 94 souches de microorganismes n'appartenant pas au genre *Mycobacterium* :
   - 54 souches de *Pseudomonas aeruginosa,*
   - 15 autres souches de bactéries non-fermentaires :
      - 1 souche *d'Achromobacter* sp1,
      - 1 souche *d'Achromobacter xylosoxidans,*
      - 1 souche d'*Acinetobacter* sp.,
      - 1 souche d'*Acinetobacter baumannii,*
      - 1 souche de *Burkholderia cepacia,*
      - 1 souche de *Burkholderia contaminans,*
      - 1 souche de *Burkholderia multivorans,*
      - 1 souche de *Burkholderia stabilis,*
      - 1 souche d'*Elizabethkingia miricola,*
      - 1 souche de *Pandoraea apista,*
      - 1 souche de *Pandoraea pnomenusa,*
      - 4 souches de *Stenotrophomonas maltophilia,*
   - 7 souches d'entérobactéries (les mêmes que celles précédemment citées),
   - 10 souches de bactéries à Gram positif (les mêmes que celles précédemment citées),
   - 8 souches de champignons/levures (les mêmes que celles précédemment citées).

Dans ce Tableau 4, les résultats sont présentés en nombre et en pourcentage de souches ayant démontré une croissance sur le milieu considéré et après 7 jours de culture.

**Tableau 4**

| | **Milieu L** | **Milieu W :** | **Milieu X** : |
|---|---|---|---|
| | | Milieu L | Milieu L |
| | | + méthanesulfonate de colistine (32 mg/L) | + méthanesulfonate de colistine (32 mg/L) |
| | | + fosfomycine (400 mg/L) | + fosfomycine (400 mg/L) |
| | | + G6P (25 mg/L) | + G6P (25 mg/L) |
| | | + Amp B (5 mg/L) | + Amp B (5 mg/L) |
| | | | + C-390 (32 mg/L) |
| *M. abscessus* (52) | 52 | 52 | 51 |
| *M. boletti* (3) | 3 | 3 | 3 |
| *M. chelonae* (34) | 34 | 34 | 34 |
| *M. immonogenum* (1) | 1 | 1 | 1 |
| *M. massiliense* (6) | 6 | 6 | 6 |
| *M. salmoniphilum* (2) | 2 | 2 | 2 |
| Total mycobactéries (98) : | 98 (100 %) | 98 (100 %) | 97 (99 %) |
| *P. aeruginosa* (54) | 54 | 0 | 0 |
| Autres bactéries non-fermentaires (15) | 14 | 9 | 6 |
| Enterobactéries (7) | 7 | 2 | 0 |
| Bactéries à Gram positif (10) | 10 | 0 | 0 |
| Champignons/Levures (8) | 8 | 3 | 0 |
| Total non-mycobactéries (94) : | 93 (99 %) | 14 (15 %) | 6 (6 %) |

### 5/ - Validation de la composition du Milieu X pour la culture et la sélection de Mycobacterium tuberculosis

Sur la base de la composition du Milieu X précédent, un milieu de culture sélectif liquide, a été formulé. Ce Milieu X liquide (sans agar) a été testé pour la détection de mycobactéries, en particulier de *Mycobacterium tuberculosis,* dans le cadre d'une analyse d'échantillons de crachat provenant de 32 patients non atteints de mucoviscidose, et de 56 patients atteints de mucoviscidose.

Pour ce faire, le système de détection microbienne automatisé BacT/ALERT® 3D (bioMérieux, France) a été utilisé. Des flacons contenant le Milieu X liquide (ou Bouillon X) ont été incubés dans l'automate de détection pendant au moins 28 jours, et comparé au réactif BacT/ALERT® MP classiquement préconisé pour la détection de mycobactéries.

La composition de ce réactif correspond schématiquement à un milieu Middlebrook 7H9 supplémenté d'acide oléique, de glycérol, de sérum albumine bovine, d'amarante et d'un cocktail d'antibiotiques (amphotéricine B, azlocilline, acide nalidixique, polymyxine B, triméthoprime et vancomycine).

Par un examen au microscope et une coloration à l'auramine, la présence de bacilles acido-alcoolo-résistants a été mise en évidence dans les 32 échantillons de crachat collectés auprès des patients non atteints de mucoviscidose. Préalablement à leur inoculation dans les flacons BacT/ALERT®, renfermant soit le bouillon BacT/ALERT® MP soit le Bouillon X, ces 32 échantillons ont été traités à la soude afin d'éliminer des espèces non mycobactériennes présentes dans les échantillons, ou au moins d'en réduire la viabilité, conformément aux méthodes standards britanniques. 64 flacons BacT/ALERT® ont ainsi été préparés puis mis à incuber à 35°C dans un automate BacT/ALERT®.

S'agissant des 56 échantillons collectés auprès des patients atteints de mucoviscidose, ceux-ci ont été soumis à deux protocoles de décontamination distincts, un traitement à l'acide et un traitement à la soude, conformes aux méthodes standards britanniques, avant leur ensemencement dans les flacons de BacT/ALERT®. 224 flacons ont ainsi été préparés :
- 56 flacons contenant le bouillon BacT/ALERT® MP, dans lequel est ensemencé un échantillon préalablement traité à l'acide,
- 56 flacons contenant le bouillon BacT/ALERT® MP, dans lequel est ensemencé un échantillon préalablement traité à la soude,
- 56 flacons contenant le Bouillon X, dans lequel est ensemencé un échantillon préalablement traité à l'acide,
- 56 flacons contenant le Bouillon X, dans lequel est ensemencé un échantillon préalablement traité à la soude.

Les résultats de ces différentes cultures sont résumés dans les Tableaux 5, 6 et 7, ci-après.

Les Tableaux 5 et 6 renseignent sur la nature des mycobactéries identifiées dans les échantillons (collectés respectivement auprès des patients non atteints de mucoviscidose et auprès des patients atteints de mucoviscidose), et le nombre de flacons dans lesquels elles ont été détectées.

Le Tableau 7 présente les temps moyens de détection (en jours) pour chacune des espèces de mycobactéries détectées et identifiées, en fonction du milieu de culture utilisé.

**Tableau 5**

| | Echantillons issus de patients non-atteints de mucoviscidose | | |
|---|---|---|---|
| | Bouillon X | BacT/ALERT® MP | Résultats combinés |
| *M. tuberculosis* | 18 | 18 | 18 |
| *M. avium* | 9 | 9 | 10 |
| *M. intracellulare* | 2 | 2 | 2 |
| *M. malmoense* | 2 | 2 | 2 |
| Nombre total de souches | 31 | 31 | 32 |
| Sensibilité | 97% | 97% | |
| Nombre de flacons contaminés par une flore non-mycobactérienne | 0 | 0 | 0 |

**Tableau 6**

| | Echantillons issus de patients atteints de mucoviscidose | | |
|---|---|---|---|
| | Bouillon X | BacT/ALERT® MP | Résultats combinés |
| *M. abscessus* | 6 | 3 | 6 |
| *M. avium* | 1 | 0 | 1 |
| Nombre total de souches | 7 | 3 | 7 |
| Sensibilité | 100% | 43 % | |
| Nombre de flacons contaminés par une flore non-mycobactérienne | 7 / 112 (6,25 %) | 33 / 112 (29,5 %) | |

**Tableau 7**

| | Bouillon X | BacT/ALERT® MP |
|---|---|---|
| *M. tuberculosis* | 14,8 | 10,8 |
| *M. avium* | 13,1 | 10,8 |
| *M. intracellulare* | 7,9 | 6,2 |
| *M. malmoense* | 18,6 | 15,7 |
| *M. abscessus* | 4,2 | 5 |

Au total, 39 isolats de mycobactéries ont été récupérés à partir des 88 prélèvements initiaux. 34 isolats ont été récupérés au moyen des flacons de réactif « standards » BacT/ALERT® MP (soit une sensibilité de 87%), contre 38 avec les flacons de Bouillon X selon l'invention (soit une sensibilité de 97%). Ces données montrent que le milieu sélectif proposé par la présente invention est relativement efficace pour la culture, l'enrichissement et l'isolement des mycobactéries.

### 6/ - Préparation et validation d'un milieu sélectif Milieu Y

### a) Composition et préparation du Milieu Y

La composition du Milieu Y est la suivante (pour environ 1600 mL de composition) :
- amidon de pomme de terre (30 g),
- asparagine (3,6 g),
- phosphate monopotassique (2,4 g),
- citrate de magnésium (0,6 g),
- sulfate de magnésium (0,24 g),
- homogénat d'oeufs entiers (1 L),
- glycérol (12 mL), et
- C-390 (51,2 mg).

Cette composition correspond à celle d'un milieu Löwenstein-Jensen, à laquelle du C-390 y est ajouté, pour obtenir concentration finale de 32 mg/L. A partir de celle-ci, le Milieu Y est préparé en suivant un procédé identique à celle d'un milieu Löwenstein-Jensen (cf. Handbook of Microbiological Media, Ronald M. Atlas, 4ème Edition, 2010).

### b) Evaluation sommaire de la fertilité et de sélectivité du Milieu Y à l'égard des mycobactéries

Le Milieu Y, formulé en tubes inclinés, a été testé pour la culture de diverses souches bactériennes :
- 1 souche de *Mycobacterium fortuitum* (inoculum calibré à 0,5 McFarland et dilué au 1/10000),
- 4 souches de *Staphylococcus aureus* (inocula calibrés à 0,5 McFarland et dilués au 1/100), et
- 3 souches de *Pseudomonas aeruginosa* (inocula calibrés à 0,5 McFarland et dilués au 1/100).

La croissance de ces souches, incubées à 37°C, a été suivie jour après jour. Les résultats obtenus, présentés en pourcentages de recouvrement de la pente, sont compilés dans le Tableau 8 ci-après.

Les résultats de ces premiers tests concordent relativement bien avec les résultats constatés avec le Milieu X, formulé sur la base d'une composition Middlebrook, à savoir : une bonne croissance de la souche mycobactérienne, une inhibition totale des souches de *S. aureus,* et une inhibition partielle avec une sélectivité différentielle à l'égard des souches de *P. aeruginosa.*

**Tableau 8**

| | Croissance (% recouvrement de la pente) | | | | | |
|---|---|---|---|---|---|---|
| | *Jour 1* | *Jour 4* | *Jour 5* | *Jour 6* | *Jour 7* | *Jour 8* |
| *M. fortuitum* (ATCC 6841-4) | 0 | 60 | 80 | 80 | 80 | 80 |
| *S. aureus* (ATCC 25923) | 0 | 0 | 0 | 0 | 0 | 0 |
| *S. aureus* (ATCC 43300) | 0 | 0 | 0 | 0 | 0 | 0 |
| *S. aureus* (NCTC 12493) | 0 | 0 | 0 | 0 | 0 | 0 |
| *S. aureus* (1108 078) | 0 | 0 | 0 | 0 | 0 | 0 |
| *P. aeruginosa* (ATCC 27853) | 98 | 98 | 98 | 98 | 98 | 98 |
| *P. aeruginosa* (08 06 075) | 0 | 2 | 2 | 2 | 5 | 20 |
| *P. aeruginosa* (08 06 058) | - | - | 25 | 25 | 25 | 25 |

Le Milieu Y, formulé en tubes inclinés, a également été testé pour la culture de souches mycobactériennes à croissance lente :
- 1 souche de *Mycobacterium tuberculosis,*
- 1 souche de *Mycobacterium bovis,* et
- 1 souche de *Mycobacterium gordonae.*

Ces tests ont été réalisés avec des inocula calibrés à 0,5 McFarland et dilués au 1/10 000.

Les résultats obtenus, présentés en pourcentages de recouvrement de la pente, sont compilés dans le Tableau 9 ci-après, et comparés à ceux d'une culture sur le milieu Löwenstein-Jensen.

**Tableau 9**

| | Croissance (% recouvrement de la pente) | | | | | |
|---|---|---|---|---|---|---|
| | *M. tuberculosis* (ATCC 25177) | | *M. bovis* (14-510617-01) | | *M. gordonae* (14-233192-01) | |
| | Löw.-Jen. | Milieu Y | Löw.-Jen. | Milieu Y | Löw.-Jen. | Milieu Y |
| *Jour 7* | 5 | 5 | 0 | 0 | 0 | 0 |
| *Jour 12* | 30 | 10 | 0 | 0 | 5 | 0 |
| *Jour 19* | 50 | 50 | 5 | 5 | 60 | 80 |
| *Jour 26* | 55 | 70 | 10 | 20 | 60 | 80 |
| *Jour 40* | 60 | 80 | 50 | 95 | 70 | 80 |

### 7/ - Données complémentaires : Sensibilité de différentes bactéries et de différentes espèces fongiques à l'égard de C-390

Une évaluation de la sensibilité à C-390 de différents microorganismes susceptibles d'infecter les voies respiratoires a été effectuée. Cette évaluation a été réalisée en utilisant le milieu W de l'exemple 4, renfermant une concentration variable de C-390._Le Tableau 10 ci-après rassemble les observations recueillies sur l'intensité de croissance des souches testées en fonction de la concentration en C-390 du milieu. Les abréviations NG (« *no growth* ») et Tr (« traces ») signifient qu'aucune croissance n'a été constatée ou seulement quelques traces peu significatives.

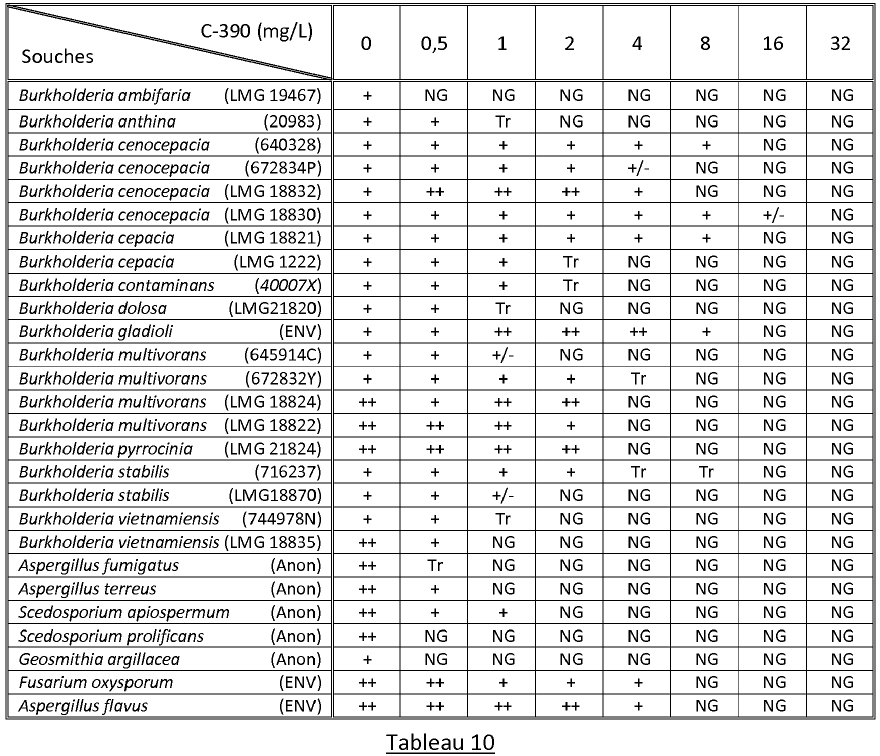

## Revendications

1. Procédé d'enrichissement et de culture sélective de mycobactéries contenues dans un échantillon biologique, dans lequel on ensemence tout ou partie dudit échantillon dans/sur un milieu de culture comprenant une composante nutritive propice au développement et à la croissance de mycobactéries,
**caractérisé en ce que** ledit milieu de culture comprend également, à titre d'agents sélectifs, au moins l'hydrochlorure de 9-chloro-9-(4'-diéthylamino)phényl-9,10-dihydro-10-phénylacridine (ou C-390) et un agent apte à inhiber des bactéries *Pseudomonas aeruginosa.*

2. Procédé selon la revendication 1, **caractérisé en ce que** la concentration en C-390 dudit milieu de culture est comprise entre 2 mg/L et 1000 mg/L.

3. Procédé selon la revendication 1 ou 2, **caractérisé en ce que** ledit agent apte à inhiber des bactéries *Pseudomonas aeruginosa* est choisi parmi : le méthanesulfonate de colistine, la fosfomycine, l'acide nalidixique, l'aztréonam, la cefsulodine et le mangrolide A.

4. Procédé selon l'une quelconque des revendications 1 à 3, **caractérisé en ce que** ledit milieu de culture comprend également un agent apte à inhiber les bactéries *Burkholderia cepacia,* préférentiellement choisi parmi la fosfomycine, l'aztréoname le mangrolide A.

5. Procédé selon l'une quelconque des revendications 1 à 4, **caractérisé en ce que** ledit milieu de culture comprend en outre, au moins un agent sélectif additionnel choisi parmi : l'amphotéricine B, l'acide nalidixique, la vancomycine, le méthanesulfonate de colistine, la fosfomycine et le vert de malachite.

6. Procédé selon la revendication 5, **caractérisé en ce que** ledit milieu de culture comprend à titre d'agents sélectifs additionnels :
- l'amphotéricine B, à une concentration comprise entre 2 mg/L et 50 mg/L ;
- le méthanesulfonate de colistine, à une concentration comprise entre 15 mg/L et 150 mg/L ;
- la fosfomycine, à une concentration comprise entre 200 mg/L et 2000 mg/L.

7. Procédé selon la revendication 6, **caractérisé en ce que** ledit milieu de culture comprend également, en complément de la fosfomycine, du glucose-6-phosphate, à une concentration comprise entre 10 mg/L et 50 mg/L.

8. Procédé selon l'une quelconque des revendications 1 à 7, **caractérisé en ce que** ledit milieu de culture comprend une composante nutritive reprenant celle d'un milieu de culture choisi parmi :
- un milieu de culture Middlebrook,
- un milieu Löwenstein-Jensen,
- un milieu Columbia,
- un milieu BCSA.

9. Procédé selon l'une quelconque des revendications 1 à 8, **caractérisé en ce que** ledit milieu de culture comprend une composante nutritive reprenant celle d'un milieu Middlebrook, complémenté avec :
- du glycérol,
- un supplément OADC,
- un ou plusieurs nutriments choisis parmi : le sang frais, un extrait de levure, l'alpha-cétoglutarate, la caséine, le pyruvate, des peptones, l'amarante, le jaune d'oeuf, de l'ARN, le polyoxyéthylène stéarate, le tyloxapol.

10. Procédé selon la revendication 9, **caractérisé en ce que** ledit milieu de culture comprend une composante nutritive reprenant celle d'un milieu Middlebrook, complémentée avec :
- du glycérol, à une concentration comprise entre 2 mL/L et 20 mL/L,
- un supplément OADC, à une concentration comprise entre 50 mL/L et 200 mL/L, et
- un extrait de levure, à une concentration comprise entre 0,1 g/L et 20 g/L.

11. Milieu de culture comprenant une composante nutritive propice au développement et à la croissance de mycobactéries et, à titre d'agents sélectifs, de l'hydrochlorure de 9-chloro-9-(4'-diéthylamino)phényl-9,10-dihydro-10-phénylacridine (ou C-390), un agent apte à inhiber des bactéries *Pseudomonas aeruginos, et* à titre d'agents sélectifs additionnels :
- l'amphotéricine B, à une concentration comprise entre 2 mg/L et 50 mg/L ;
- le méthane sulfonate de colistine, à une concentration comprise entre 15 mg/L et 150 mg/L;
- la fosfomycine, à une concentration comprise entre 200 mg/L et 2000 mg/L, et du glucose-6-phosphate, à une concentration comprise entre 10 mg/L et 50 mg/L.

12. Milieu de culture selon la revendication 11, **caractérisé en ce qu'**il comprend une composante nutritive reprenant celle d'un milieu de culture choisi parmi :
- un milieu Middlebrook,
- un milieu Löwenstein-Jensen,
- un milieu Columbia,
- un milieu BCSA.

13. Milieu de culture selon la revendication 11 dans lequel, l'hydrochlorure de 9-chloro-9-(4'-diéthylamino)phényl-9,10-dihydro-10-phénylacridine est à une concentration comprise entre 2 mg/L et 1 000 mg/L, et ladite composante nutritive propice au développement et à la croissance de mycobactéries consiste en un milieu Middlebrook 7H9, complémenté avec :
- du glycérol, à une concentration comprise entre 2 mL/L et 20 mL/L,
- un supplément OADC, à une concentration comprise entre 50 mL/L et 200 mL/L, et
- un extrait de levure, à une concentration comprise entre 0,1 g/L et 20 g/L.

## Patentansprüche

1. Verfahren zur Anreicherung und zur selektiven Kultur von in einer biologischen Probe enthaltenen Mykobakterien, wobei man die gesamte oder einen Teil der Probe in/auf ein Kulturmedium überimpft, das eine die Entwicklung und das Wachstum von Mykobakterien begünstigende Nährkomponente umfasst,
**dadurch gekennzeichnet, dass** das Kulturmedium außerdem als Selektionsmittel mindestens 9-Chlor-9-(4'-diethylamino)phenyl-9,10-dihydro-10-phenylacridinhydrochlorid (oder C-390) und ein Mittel, das zur Hemmung von *Pseudomonas aeruginosa*-Bakterien geeignet ist, umfasst.

2. Verfahren nach Anspruch 1, **dadurch gekennzeichnet, dass** die C-390-Konzentration des Kulturmediums zwischen 2 mg/l und 1000 mg/l beträgt.

3. Verfahren nach Anspruch 1 oder 2, **dadurch gekennzeichnet, dass** das Mittel, das zur Hemmung von *Pseudomonas aeruginosa*-Bakterien geeignet ist, ausgewählt ist aus: Colistinmethansulfonat, Fosfomycin, Nalidixinsäure, Aztreonam, Cefsulodin und Mangrolid A.

4. Verfahren nach einem der Ansprüche 1 bis 3, **dadurch gekennzeichnet, dass** das Kulturmedium außerdem ein Mittel umfasst, das zur Hemmung von *Burkholderia* cepacia-Bakterien geeignet ist, vorzugsweise ausgewählt aus Fosfomycin, Aztreonam, Mangrolid A.

5. Verfahren nach einem der Ansprüche 1 bis 4, **dadurch gekennzeichnet, dass** das Kulturmedium außerdem mindestens ein zusätzliches Selektionsmittel umfasst, ausgewählt aus: Amphotericin B, Nalidixinsäure, Vancomycin, Colistinmethansulfonat, Fosfomycin und Malachitgrün.

6. Verfahren nach Anspruch 5, **dadurch gekennzeichnet, dass** das Kulturmedium als zusätzliche Selektionsmittel Folgende umfasst:
- Amphotericin B in einer Konzentration zwischen 2 mg/l und 50 mg/l:
- Colistinmethansulfonat in einer Konzentration zwischen 15 mg/l und 150 mg/l;
- Fosfomycin in einer Konzentration zwischen 200 mg/l und 2000 mg/l.

7. Verfahren nach Anspruch 6, **dadurch gekennzeichnet, dass** das Kulturmedium zusätzlich zu Fosfomycin außerdem Glucose-6-phosphat in einer Konzentration zwischen 10 mg/l und 50 mg/l umfasst.

8. Verfahren nach einem der Ansprüche 1 bis 7, **dadurch gekennzeichnet, dass** das Kulturmedium eine Nährkomponente umfasst, die diejenige eines Kulturmediums aufgreift, ausgewählt aus:
- einem Middlebrook-Kulturmedium,
- einem Löwenstein-Jensen-Medium,
- einem Columbia-Medium,
- einem BCSA-Medium.

9. Verfahren nach einem der Ansprüche 1 bis 8, **dadurch gekennzeichnet, dass** das Kulturmedium eine Nährkomponente umfasst, die diejenige eines Middlebrook-Mediums aufgreift, angereichert mit:
- Glycerin,
- einer OADC-Anreicherung,
- einem oder mehreren Nährstoffen, ausgewählt aus: frischem Blut, einem Hefeextrakt, alpha-Ketoglutarat, Casein, Pyruvat, Peptonen, Amarant, Eigelb, RNA, Polyoxyethylenstearat, Tyloxapol.

10. Verfahren nach Anspruch 9, **dadurch gekennzeichnet, dass** das Kulturmedium eine Nährkomponente umfasst, die diejenige eines Middlebrook-Mediums aufgreift, angereichert mit:
- Glycerin in einer Konzentration zwischen 2 ml/l und 20 ml/l,
- einer OADC-Anreicherung in einer Konzentration zwischen 50 ml/l und 200 ml/l
und
- einem Hefeextrakt in einer Konzentration zwischen 0,1 g/l und 20 g/l.

11. Kulturmedium, umfassend eine die Entwicklung und das Wachstum von Mykobakterien begünstigende Nährkomponente und als Selektionsmittel mindestens 9-Chlor-9-(4'-diethylamino)phenyl-9,10-dihydro-10-phenylacridinhydrochlorid (oder C-390), ein Mittel, das zur Hemmung von *Pseudomonas* aeruginosa-Bakterien geeignet ist, und als zusätzliche Selektionsmittel:
- Amphotericin B in einer Konzentration zwischen 2 mg/l und 50 mg/l;
- Colistinmethansulfonat in einer Konzentration zwischen 15 mg/l und 150 mg/l;
- Fosfomycin in einer Konzentration zwischen 200 mg/l und 2000 mg/l sowie Glucose-6-phosphat in einer Konzentration zwischen 10 mg/l und 50 mg/l.

12. Kulturmedium nach Anspruch 11, **dadurch gekennzeichnet, dass** es eine Nährkomponente umfasst, die diejenige eines Kulturmediums aufgreift, ausgewählt aus:
- einem Middlebrook-Kulturmedium,
- einem Löwenstein-Jensen-Medium,
- einem Columbia-Medium,
- einem BCSA-Medium.

13. Kulturmedium nach Anspruch 11, wobei 9-Chlor-9-(4'-diethylamino)phenyl-9,10-dihydro-10-phenylacridinhydrochlorid in einer Konzentration zwischen 2 mg/l und 1000 mg/l vorliegt und die Nährkomponente, die die Entwicklung und das Wachstum von Mykobakterien begünstigt, aus einem Middlebrook 7H9-Medium besteht, angereichert mit:
- Glycerin in einer Konzentration zwischen 2 ml/l und 20 ml/l,
- einer OADC-Anreicherung in einer Konzentration zwischen 50 ml/l und 200 ml/l
und
- einem Hefeextrakt in einer Konzentration zwischen 0,1 g/l und 20 g/l.

## Claims

1. A process for the enrichment and selective culture of mycobacteria contained in a biological sample, wherein all or part of said sample is inoculated in/on a culture medium comprising a nutritive component suitable for the development and growth of mycobacteria,
**characterized in that** said culture medium also comprises, as selective agents, at least 9-chloro-9-(4'-diethylamino)phenyl-9,10-dihydro-10-phenylacridine hydrochloride (or C-390) and an agent capable of inhibiting *Pseudomonas aeruginosa* bacteria.

2. The process as claimed in claim 1, **characterized in that** the C-390 concentration of said culture medium is between 2 mg/l and 1000 mg/L

3. The process as claimed in claim 1 or 2, **characterized in that** said agent capable of inhibiting *Pseudomonas aeruginosa* bacteria is chosen from: colistin methanesulfonate, fosfomycin, nalidixic acid, aztreonam, cefsulodine and mangrolide A.

4. The process as claimed in any one of claims 1 to 3, **characterized in that** said culture medium also comprises an agent capable of inhibiting *Burkholderia cepacia* bacteria, preferentialy chosen from fosfomycin, aztreonam and mangrolide A.

5. The process as claimed in any one of claims 1 to 4, **characterized in that** said culture medium also comprises at least one additional selective agent chosen from: amphotericin B, nalidixic acid, vancomycin, colistin methanesulfonate, fosfomycin and malachite green.

6. The process as claimed in claim 5, **characterized in that** said culture medium comprises, as additional selective agents:
- amphotericin B, at a concentration of between 2 mg/l and 50 mg/l;
- colistin methanesulfonate, at a concentration of between 15 mg/l and 150 mg/l;
- fosfomycin, at a concentration of between 200 mg/l and 2000 mg/l.

7. The process as claimed in claim 6, **characterized in that** said culture medium also comprises, in addition to the fosfomycin, glucose-6-phosphate, at a concentration of between 10 mg/l and 50 mg/L

8. The process as claimed in any one of claims 1 to 7, **characterized in that** said culture medium comprises a nutritive component reproducing that of a culture medium chosen from:
- a Middlebrook culture medium,
- a Löwenstein-Jensen medium,
- a Columbia medium,
- a BCSA medium.

9. The process as claimed in any one of claims 1 to 8, **characterized in that** said culture medium comprises a nutritive component reproducing that of a Middlebrook medium, supplemented with:
- glycerol,
- an OADC supplement,
- one or more nutrients chosen from: fresh blood, a yeast extract, alpha-ketoglutarate, casein, pyruvate, peptones, amaranth, egg yolk, RNA, polyoxyethylene stearate and tyloxapol.

10. The process as claimed in claim 9, **characterized in that** said culture medium comprises a nutritive component reproducing that of a Middlebrook medium, supplemented with:
- glycerol, at a concentration of between 2 ml/l and 20 ml/l,
- an OADC supplement, at a concentration of between 50 ml/l and 200 ml/l, and
- a yeast extract, at a concentration of between 0.1 g/l and 20 g/l.

11. A culture medium comprising a nutritive component suitable for the development and growth of mycobacteria and, **characterized in that** it also comprises, as selective agents, 9-chloro-9-(4'-diethylamino)phenyl-9,10-dihydro-10-phenylacridine hydrochloride (or C-390) and an agent capable of inhibiting *Pseudomonas aeruginosa* bacteria, and as additional selective agents:
- amphotericin B, at a concentration of between 2 mg/l and 50 mg/l:
- colistin methanesulfonate, at a concentration of between 15 mg/l and 150 mg/l;
- fosfomycin, at a concentration of between 200 mg/l and 2000 mg/l, and glucose-6-phosphate, at a concentration of between 10 mg/l and 50 mg/l.

12. The culture medium as claimed in claims 1, **characterized in that** it comprises a nutritive component reproducing that of a culture medium chosen from:
- a Middlebrook medium,
- a Löwenstein-Jensen medium,
- a Columbia medium,
- a BCSA medium.

13. The culture medium as claimed in claim 11, wherein 9-chloro-9-(4'-diethylamino)phenyl-9,10-dihydro-10-phenylacridine hydrochloride is at a concentration of between 2 mg/l and 1,000 mg/l and said nutritive component suitable for the development and growth of mycobacteria consists of Middlebrook 7H9 medium, supplemented with:
- glycerol, at a concentration of between 2 ml/l and 20 ml/l.
- an OADC supplement, at a concentration of between 50 ml/l and 200 ml/l, and
- a yeast extract, at a concentration of between 0.1 g/l and 20 g/l.
